# EUROPEAN PATENT APPLICATION

(11) **EP 2 028 454 A1**
(43) Date of publication of application: **25.02.2009**
(21) Application number: 07711057.5
(22) Date of filing: 07.03.2007
(51) Int. Cl.: G01D 21/02, G01N 27/00, G01N 25/00

(54) **AN ENVIRONMENTAL MONITORING APPARATUS AND METHOD THEREOF**

(30) Priority: 10.03.2006 CN 200610057261
(71) Applicant: Law, Sui-Chun, Tin Shui Wai, N.T. Hong Kong (CN); Chan, Yiu Wai, N.T., Hong Kong (CN)
(72) Inventor: Law, Sui-Chun, Tin Shui Wai, N.T. Hong Kong (CN); Chan, Yiu Wai, N.T., Hong Kong (CN)
(74) Representative: Vogel, Andreas
(86) International application number: PCT/CN2007/000736
(87) International publication number: WO 2007/104240

(57) **Abstract**

The present invention is related to an environmental monitoring and analyzing device. The said device contains sensors, control unit and display unit. The said sensors detect the values of different environmental parameters. The control unit of the device received the values from the sensors. By judging the values against the pre-set built-in standards and criteria, the parameter ranges are defined. The implications of the corresponding judged results of the said parameter ranges are then output and being displayed on the display unit. The present invention offers a method for environmental monitoring and analyzing. By implementing this invention into an embodiment, the environmental monitoring analyzing and data adjustment can be carried out instantly by inter-correlating the levels of different sensing parameters simultaneously. The analyzed results are therefore much more accurate. A real-time comprehensive and easily understood air quality report can could be provided

## Description

### Field of the Invention

The present invention is related to the technology for environmental monitoring. More specifically, it is related to the device and the method for environmental monitoring and analyzing.

### Background of the invention

As the problems of indoor air pollution are getting severe, the public's concerns on their living and working environment as well as the health effects by the indoor air quality are getting increasing. At the same time, the guidelines and the rules to control and regulate the indoor air quality are established in different countries. Hence, the demand on instruments and equipment for monitoring the air quality is increasing.

Conventionally, there are two major types of instruments for monitoring the indoor air quality. The first type of environmental monitoring instruments being employed mainly in the research laboratories. They are of considerably large scale. The second type belongs to the portable survey type instruments and they are much more compact in size.

The results obtained by the first type of environmental monitoring instrument are highly precise and accurate. Nevertheless, the prices of this type of instruments are expensive. The operations of these instruments are complicated and only manageable by trained and skillful technicians.

Generally, each second type environmental monitoring instrument is equipped with a sensor for measuring a particular environmental parameter. The sizes of this type of instrument are therefore comparatively compact. Nevertheless, as different environmental parameters are inter-correlated, the level of a single parameter is usually affected by the other parameters. To obtain the level of a particular environmental parameter with a single sensor is usually not an accurate method. The precision obtained would be low. For examples, to measure the concentration of the volatile organic compounds merely by the photo-ionization detection method could gave inaccurate result as the detection method is easily affected by the temperature and relative humidity of the environment. In addition, different types of sensors with different working principles give different outcomes if they are employed for monitoring the same environmental parameter. For these reasons, there are usually difficulties to standardize the detection methods for the environmental parameters. In order to ensure an adequate and a moderately accurate result be obtained for a single parameter, several instruments are usually brought to site during measurement. The results obtained are then evaluated together during analyzing. The measurement processes by multiple instruments are rather inconvenience.

Nevertheless, for all types of environmental monitoring instruments being mentioned, only the raw data would be simply displayed and output. None of them would provides systematic analyzed information as describes in the present invention.

### Summary of the Invention

The present invention has a major object to solve the deficiencies by the current environmental monitoring devices and methods. It has the further object to provide a device and a method for environmental monitoring and analyzing. Different levels of the environmental parameters are detected and measured at the same time. They are then being judged and analyzed systematically. A real time and comprehensive air quality report will be generated. The construction of the device is simple. It is easy to be operated even by the non-technical users. The environmental parameters evaluated are highly accurate and precise.

The present invention provides a device to monitor the environment, and to solve the problems by the conventional environmental monitoring instruments. The device contains sensors, control unit and display unit. The said sensors obtain the values of different environmental parameters. The values are then sent to the control unit for further analysis. By judging the value of each measured parameter against the pre-set built-in standards and criteria, the ranges of some environmental parameters are the defined. The implications of the said ranges of some environmental parameters are then output and being displayed onto the display unit.

The said standards and criteria for judging the values of the environmental parameters include a first judgment principle. The said first judgment principle defines more than one parameter ranges for the value of each measured environmental parameter. There are implications, which included the messages of recommendations, for the value of the measured environmental parameter which fall into the said parameter ranges.

The said standards and criteria for environmental parameters include a second judgment principle. The said second judgment principle defines one or more than one conditional array. One or more than one parameter ranges defined by the first judgment principle congregate and become the prerequisite parameter ranges for the establishment of each said conditional array. The implications of each said conditional array include some messages of the potential problems.

Besides, the said implications also include some messages of the recommendations subjecting to the said potential problems.

The said standards and criteria for environmental parameters include a third judgment principle. The said third judgment principle defines more than one categories for each measured environmental parameter. Air-quality-level judgment standards for air quality levels would be defined based on the combination of different categories of the measured environmental parameters. The implications include the messages of the corresponding air quality level by the said air-quality-level judgment standards.

The said sensor could be any sensor of the temperature, relative humidity, volatile organic compounds, carbon monoxide, carbon dioxide, dust, ozone, carbon dioxide, air flow rate, radon, formaldehyde.

The said control unit contains power supply and control circuit, input circuits, the central processing unit, the memory that stored the pre-set built-in standards and criteria for judging the environmental parameters as well as the implications and corresponding messages, and the output circuit. The power supply and control circuit connects the external power supply to the device. The input circuit collects the values from the sensors of different environmental parameters, and output them to the central processing unit. The central processing unit analyzes the input signals base on the pre-set built-in standards and criteria and defines the parameter ranges of each environmental parameter. The implications of each said environmental range are then output and being displayed onto the display unit.

The said input circuit includes analog and digital converter and low pulse timer.

The present invention also offers a method to monitor and analyze the environment. The respective steps are as follow:

Judge the value of the environmental parameters against pre-set built-in standard and criteria. Define the parameter ranges of the measured environmental parameters. The implications corresponding to the said parameter ranges are obtained; and

Output the parameter ranges and the implications obtained.

The said pre-set built-in standard and criteria includes the first judgment principle, the second judgment principle and the third judgment principle. The said first judgment principle defines parameter ranges for the value of each measured environmental parameter. The said second judgment principle defines the conditional arrays. One or more than one parameter ranges defined by the first judgment principle congregate and become the prerequisite parameter ranges for the establishment of each said conditional array. The third judgment principle defines more than one categories for each measured environmental parameter Air quality level by the air-quality-level judgment standards would be defined based on the combination of different categories of the measured environmental parameters. The said implications included the messages of the recommendations corresponding to the said parameter ranges, the messages of the potential problems corresponding to the said conditional arrays, the messages of the recommendations corresponding to the said potential problems, and the messages of the corresponding air quality level by the said air-quality-level judgment standards.

The said environmental parameters include any parameter of the temperature, relative humidity, volatile organic compounds, carbon monoxide, carbon dioxide, dust, ozone, carbon dioxide, airflow rate, radon, formaldehyde.

In the present invention, the values of different environmental parameters by different sensors are obtained. Real time data adjustments and analyses are performed on the values, based on the inter-correlation between the different environmental parameters. More accurate and precise results and information are then presented. A comprehensive and an easy-to-understand air quality report will be generated simultaneously. (Wherein, the report includes the implications regarding the potential problems, the recommendations and the message corresponding to the air quality level.) For certain environmental parameters which need longer testing time by conventional methods, such as the parameters of airborne bacteria and fungus which need hours for bacteria and fungus incubations by the conventional methods, the present invention would able to provides instant level assessment by means of forecasting, based on the inter-correlationship between different measured environmental parameters. For instance, in a warm and humid environment where the dust level is attained at certain high level (in an environment where the level of respirable suspended particulates is high), the pre-requisite conditions for growing and incubating the airborne bacteria are actually created. Based on the values of the temperature, relative humidity and level of respirable suspended particulates, the level of airborne bacteria can then be forecasted simultaneously. On another example, in an environment where the concentration of the carbon dioxide is sustained at high level, poor ventilation or too much occupants are implied. With the present invention, the implications of the environment would be generated. These could be the messages of recommendations such as "turn on the air exhausting system", "decrease the number of occupants" and "open the windows" etc. The device by the present invention is structurally simple and low cost. The device is easy to be handled by non-technical users.

The following figures and description review the further details of the present invention.

### Brief Description of the Drawings

Figure 1 indicates circuit modules of the environmental device of the present invention
Figure 2 indicates the block circuit diagram of the environmental device of the present invention
Figure 3 indicates the circuit diagram for temperature sensor in the environmental device of the present invention
Figure 4 indicates the circuit diagram for relative humidity sensors in the environmental device of the present invention
Figure 5 indicates the circuit diagram for volatile organic compounds sensors in the environmental device of the present invention
Figure 6 indicates the circuit diagram for carbon monoxide sensors in the environmental device of the present invention
Figure 7 indicates the circuit diagram for carbon dioxide sensors in the environmental device of the present invention
Figure 8 indicates the circuit diagram for dust sensors in the environmental device of the present invention

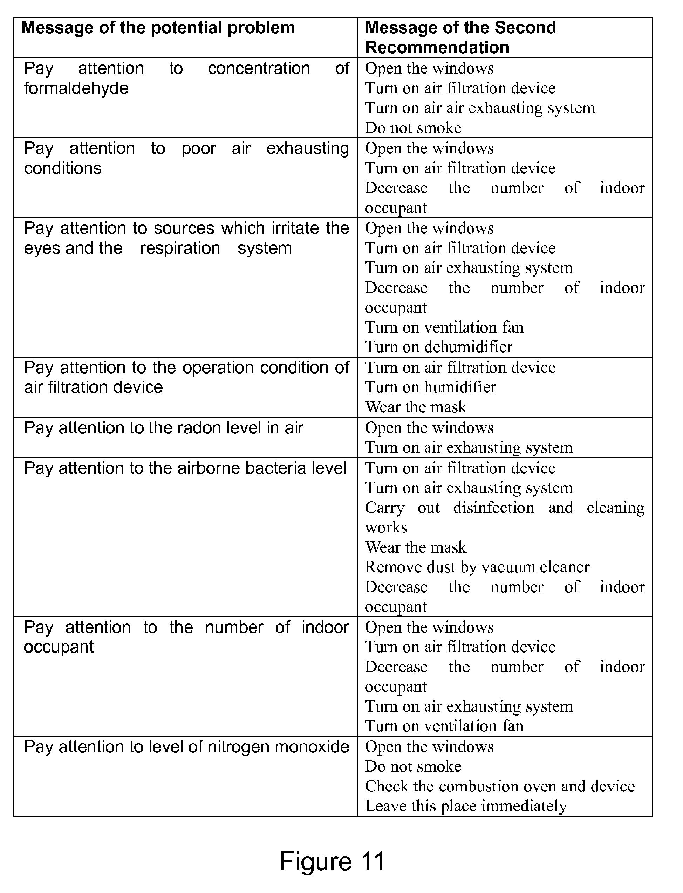

Figures 9-13 indicate the examples on the parameter judgment standards and criteria, as well as the resulted implications
Figure 14 indicates the flowchart of the environmental monitoring and analyzing by the present invention

### Detailed Description of the Drawings

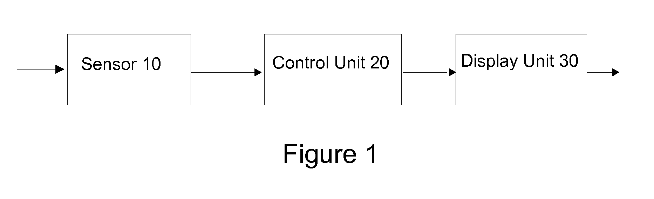

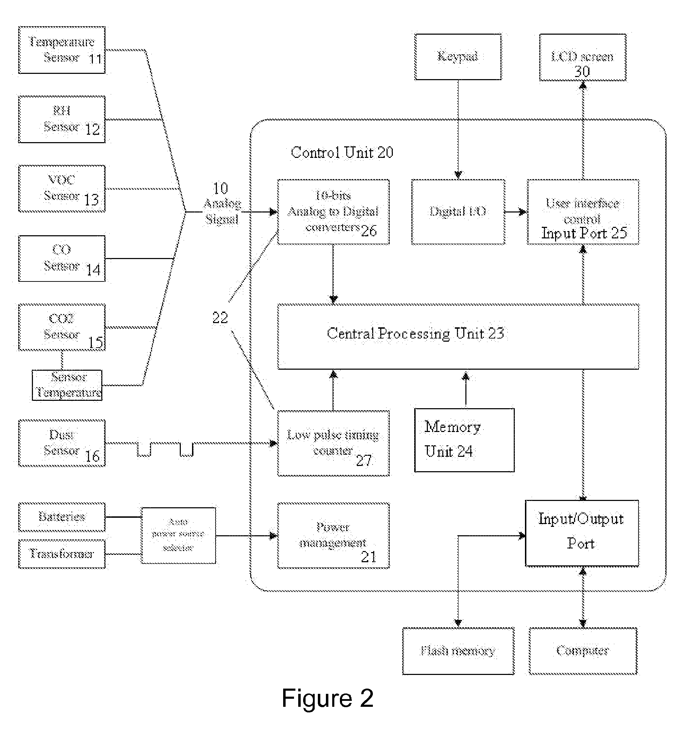

As indicated in Figure 1 and 2, the device of the present invention contains the sensors 10, the control unit 20 and the display unit 30.

The said sensors 10 are employed to obtain the values of different environmental parameters. The values obtained are input and be sent to the control unit 20. In the present embodiment, the sensors 10 employed included the temperature sensor, the relative humidity sensor 12, the volatile organic compound sensor 13, the carbon monoxide sensor 14, the carbon dioxide sensor 15, and the dust sensor 16. Nonetheless, others environmental sensors such as the ozone sensor, the nitrogen dioxide sensor, the air flow rate sensor, the radon level sensor and the formaldehyde sensor can also be included for same working purpose.

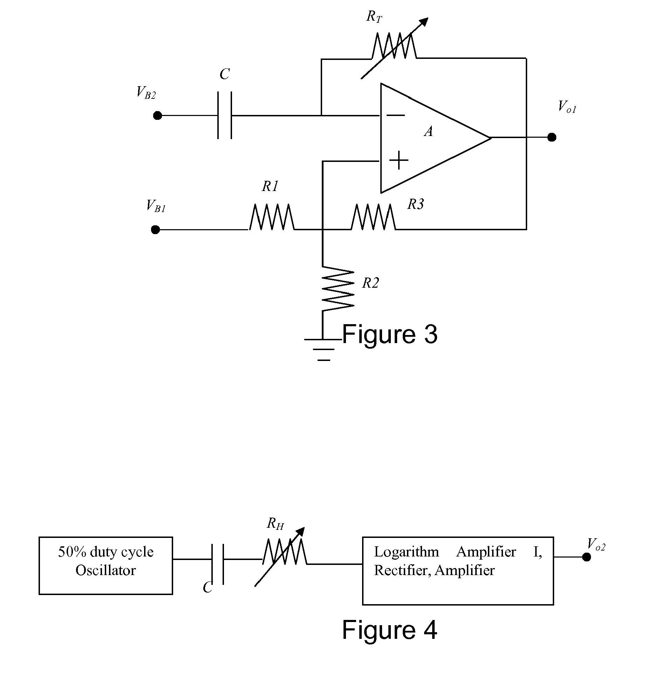

Figures 3-8 indicate the circuit diagrams for the sensors in the embodiment of the present invention. The circuit for the temperature sensor 11 is shown in Figure 3. In the present embodiment, a thermistor which its resistance varies with the temperature is employed as the temperature sensor. The change of temperature in the environment changes the resistance of the thermistor R_{T}. The change of value of thermistor R_{T} is represented by a voltage and being output. The output voltage Vol of is sent to the control unit 20. The output of the temperature sensor belongs to a train of periodic signals, whereas the frequencies of the periodic signals are temperature dependent. The control unit 20 detects the frequency of the waveform and determines the measured temperature.

Figure 4 indicates the circuit for relative humidity sensor 12 in the present embodiment. In the present embodiment, the relative humidity sensor 12 belongs to the resistive type relative humidity sensor is employed. A capacitor C is connected in series to a humidity sensitive resistor R_{H}. After being amplified and with blocking out the DC component, the signal is output as voltage. The said circuit is effective to block off the entire DC component and protect the humidity sensitive resistor R_{H}. It is a simple circuit and adaptive to different duty cycles of the input signals. As indicates in the figure, a 50% oscillation duty cycle is employed.

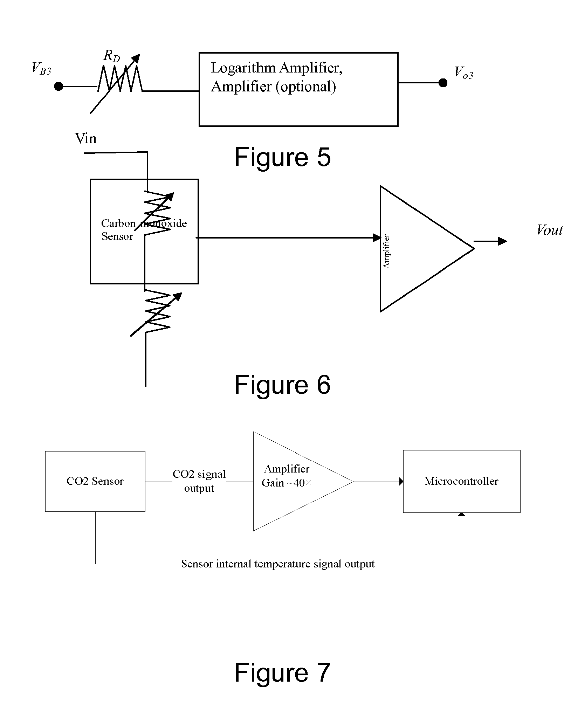

Figure 5 indicates the circuit for the sensor of volatile organic compounds 13 in the present embodiment. In the present embodiment, the sensor of volatile organic compounds 13 belongs to heated metal oxides type. The sensor varies its resistance R_{D} with the concentration of volatile organic compounds. The input voltage V_{B3} would first go through the resistor with resistance R_{D}, it will then be amplified by an analog amplifier. The voltage output is then sent to the control unit.

Figure 6 indicates the circuit for the carbon monoxide sensor 14 in the present embodiment. In the present embodiment, the carbon monoxide sensor 14 being employed belongs to the heated metal oxide type sensor. The sensor varies its resistance with the concentration of carbon monoxide. The input voltage would first go through the resistor with resistance, it will then be amplified by an analog amplifier. The voltage output is then sent to the control unit.

Figure 7 indicates the circuit of carbon dioxide sensor 15 in the present embodiment. In the present embodiment, the carbon dioxide sensor 15 belongs to the heated metal oxide type. A heating element is included in addition to the sensor element. The resistance of the sensor changes with the concentration of carbon dioxide. The input voltage would first go through the resistor with resistance, it will then be amplified by an analog amplifier and be sent to the control unit 20. In order to obtain an accurate value for carbon dioxide, the desired operation temperature of the sensor is maintained by the built-in heater. The influence of the environmental temperature and ambient carbon dioxide can be eliminated by comparing the voltage output obtained with that of the ambient air. Hence, a more accurate result could be obtained. In addition, the internal temperature of the sensor by the heating element would be fed to control unit 20. This is acts as a reference for showing that the sensor has been warmed-up, and indicating that sensor reached the optimal operation temperature.

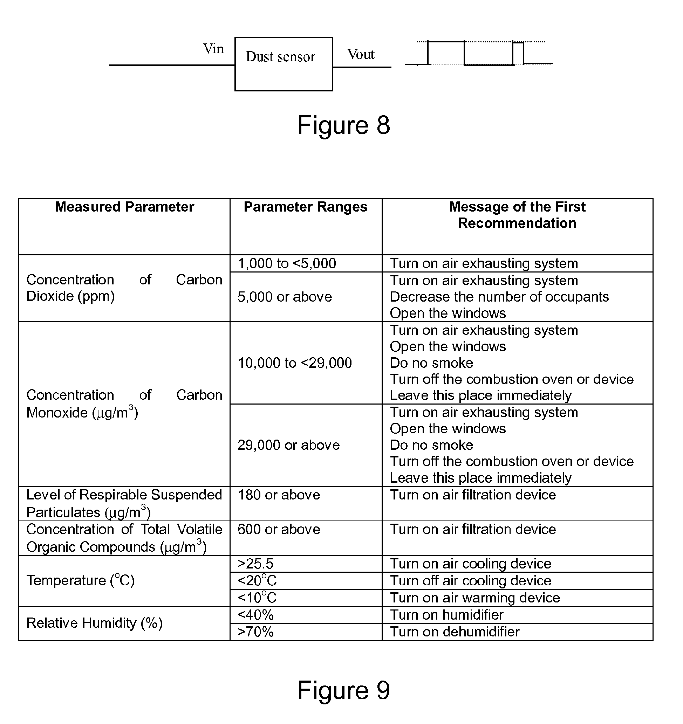

Figure 8 indicates the circuit for the dust sensor 16 in the present embodiment. In the present embodiment, the dust sensor 16 belongs to the light scattering type sensor. The output of dust sensor will go to low voltage (ground level) when the particulate matters are detected, otherwise the output will state at high voltage. In another word, the low pulse occupancy time is proportional to dust concentration. By obtaining the ratio of the time of total low pulse and high pulse, the control unit 20 would able to calculate the corresponding dust level.

The control unit 20 in the present embodiment includes a power supply and control circuit 21, a voltage input circuit 22, a central processing unit 23, a memory unit 24 and a voltage output circuit 25. The power supply and control circuit 21 obtains the power from the external power supply. It could be either AC or DC power supply. The auto power source selector will switch the power source to transformer when the power plug is inserted.

The voltage input circuit 22 receives the measured values from the sensors 10. In the present embodiment, an analog to digital converter 26 and a low pulse time counter 27 are included in the voltage input circuit 22. The analog to digital converter 26 received the signals from the temperature sensor 11, the relative humidity sensor 12, the volatile organic compounds sensor 13, the carbon monoxide sensor 14, and the carbon dioxide sensor 15, as well as the reference signals by the carbon dioxide sensor 15. The analogue signals are then converted to digital signals and be input to the central processing unit 23. The low pulse time counter 27 is used to obtain the input signal from the dust sensor circuit. An average value of low pulse timing obtained from dust sensor circuit is calculated and be sent to the central processing unit 23. Nonetheless, the voltage input circuit described is based on the types of sensors being employed in the present embodiment. The voltage input circuit can be modified to fit the different type sensors in different devices and embodiments.

The memory unit 24 stored the first judgment principle, the second judgment principle and the third judgment principle, as well as the implications referring to the results obtained after the judgments.

The first judgment principle defines the parameter ranges for the value of each measured environmental parameter. The said values of measured environmental parameter is referred to that obtained by the sensors 10, such as the values obtained by the temperature sensor, the relative humidity sensor, the volatile organic compounds sensor, the carbon monoxide sensor, the carbon dioxide sensor and the dust sensor in the present embodiment. For example, the parameter ranges for the temperature could be referred to the ranges of >25.5°C, <20°C and <10°C etc. The said second judgment principle defines the conditional arrays. One or more than one parameter ranges defined by the first judgment principle congregate and become the prerequisite parameter ranges for the establishment of each said conditional array. For example, in, the parameter range for the temperature in one scenario could be defined as 25.5-35°C and the parameter range for the volatile organic compounds could be defined as above 600µg/m³. The parameter ranges for different conditional arrays could be the same range or difference. The third judgment principle defines more than one categories for each measured environmental parameter. The air quality level by the air-quality-level judgment standards would be defined based on the combination of different categories of the measured environmental parameters.

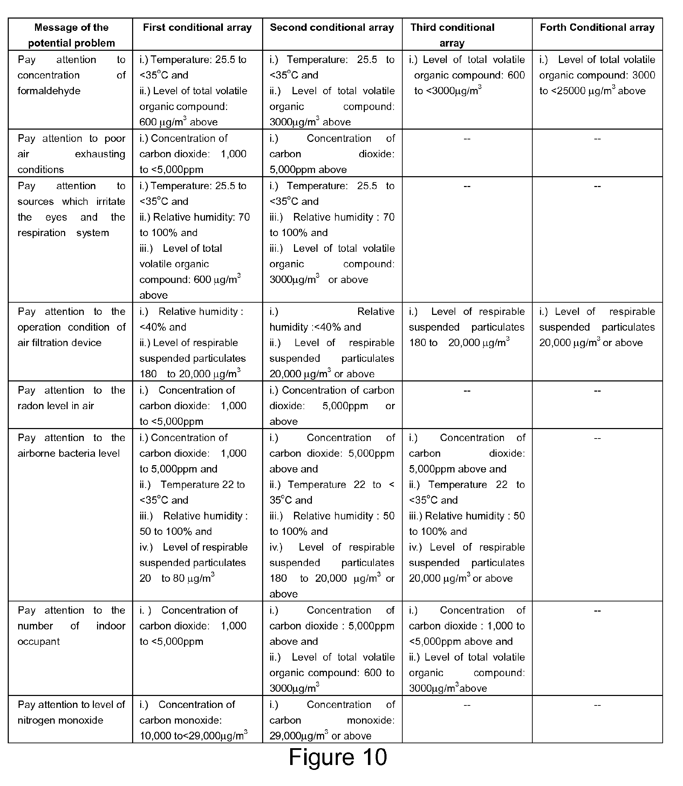

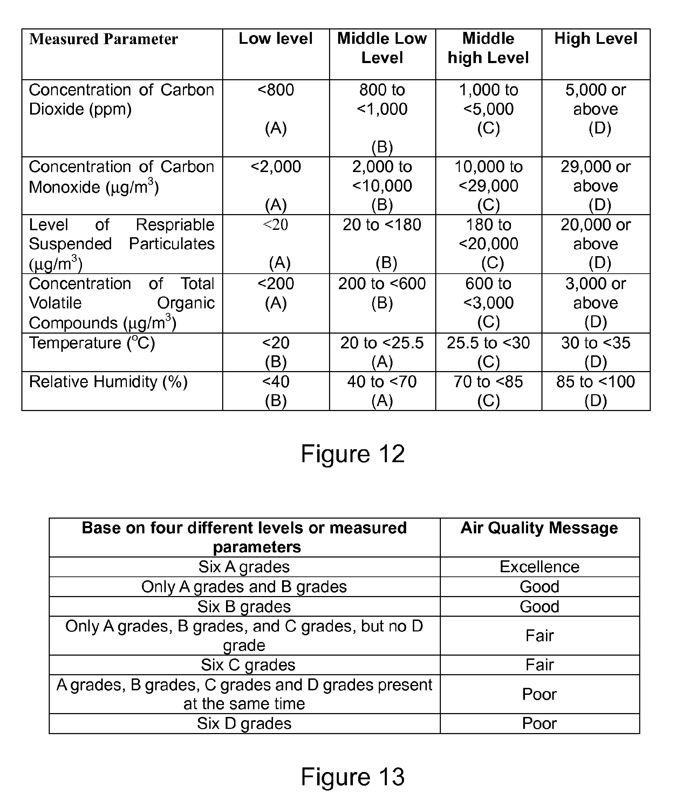

The said implications included first, the messages of the recommendations corresponding to the said parameter ranges, and second, the messages of the potential problems corresponding to the said conditional arrays and the messages of the recommendations corresponding to the said potential problems, and the messages of the corresponding air quality level by the said air-quality-level judgment standards. The first implications on the said recommendations are corresponding to the parameter ranges, which are defined by the first judgment principle. For example, as indicates in Figure 9, when the parameter range of temperature was defined as >25.5°C, the recommendation shall be "Turn on air cooling devices". The implications on the said potential problems are corresponding to the conditional arrays, which are defined by the second judgment principle based on the different parameter ranges. As shown in Figure 10, for example, when the temperature was defined with a parameter range of 25.5-35°C and the level of the total volatile organic compounds was defined with a parameter range of above 600µg/m³, the potential problem implied shall be "high level of formaldehyde". The recommendations subjected to the said potential problem shall be "open the windows", "Turn on air filtration device", "Turn on air exhausting system", "Do not smoke". The air quality level defined by the air-quality-level judgment standards based on the third judgment principle shall then be done by referring to that shown in Figure 12 and Figure 13.

The central processing unit 23 received the signals from the voltage input circuit 22, whereas they are the converted digital signals from the sensors 20 circuit. The signals are then judged against with the pre-set built-in standards and criteria in the memory unit 24. The parameter ranges of the measured environmental parameters based on the first judgment principle are then defined. Base on the correlation between the implications and the defined parameter ranges, the first recommendations are confirmed. The signals are also judged against with the pre-set build-in standards and criteria in the memory unit 24 under the second judgment principle. The said second judgment principle defines the conditional arrays. One or more than one said parameter ranges which are defined by the first judgment principle congregate and become the prerequisite parameter ranges for the establishment of each said conditional array. Based on the correlations between the implications of the potential problems and the conditional arrays, the second recommendations are then provided. The signals are also judge against with the pre-set built-in standard and criteria in the memory unit 24 under the third judgment principle. A category for each measured environmental parameter is then defined. The corresponding air quality level by the air-quality-level judgment standards are defined based on the combination of different categories of the measured environmental parameters. Based on the correlations between the implications and the air quality level, the messages for the corresponding air quality level would be confirmed by the central processing unit 23. The messages together with individual measured parameter values, would be output by the voltage output circuit 25, and be displayed in the displayed unit 30.

The display unit 30 receives the measured results and the implications, and displays them in any specific formats, wordings, numerical, and graphical characters.

The device of the present invention contains input ports and input/output ports, whereas the input ports received input signal from the keypad. The input/output ports transfer the information to other devices, such as computer, pocket size personal computer and flash memory. The input/output ports can also be connected to other devices by infra-red interface device, Bluetooth interface device and other wireless interface device.

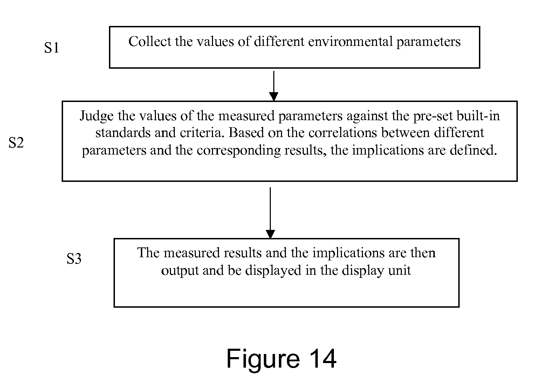

Figure 14 indicates the method of environmental monitoring and analyzing by the present invention. The sensors S 1 are employed in the first place to collect the values of different environmental parameters. The values are then sent to the control unit. The control unit in S2 would then judges the values of the measured parameters against the pre-set built-in standards and criteria. The parameters ranges of each measured environmental parameters are then defined based on the judgment principles. Based on the correlations between different parameters and the corresponding results, the implications are defined. The measured results and the implications are then output and be displayed in the display unit, as indicates in S3. The said first judgment principle defines parameter ranges for the value of each measured environmental parameter. The said second judgment principle defines the conditional arrays. One or more than one parameter ranges defined by the first judgment principle congregate and become the prerequisite parameter ranges for the establishment of each said conditional array. The third judgment principle defines more than one categories for each measured environmental parameter. The air quality level by the air-quality-level judgment standards would be defined based on the combination of different categories of the measured environmental parameters. The said implications to be displayed included the message of the first recommendations corresponding to the parameter ranges, the message of the potential problems corresponding to the conditional arrays, the second recommendations corresponding to the said potential problems, and the messages of the air quality level which is defined by the air-quality-level judgment standards.

## Claims

1. An environmental monitoring device, the said device is equipped with different type of sensors, control unit and display unit. The said different type of sensor obtained the values of different environmental parameters, the values of the measured parameters are then input to the control unit. The said control unit receives the values from the sensors and judges them against the pre-set built-in standard and criteria, the parameters ranges are defined. The implications corresponding to the judged results of the parameter ranges are output and be displayed to the display unit.
Wherein, the environmental monitoring devices carries out analysis and real time data adjustment toward different environmental parameters based on their inter-correlationships. An easy-to-understand air quality report is offered instantly.

2. An environmental monitoring device according to Claim 1, wherein, the said pre-set built-in standard and criteria includes the first judgment principle. The first judgment principle defines more than one parameter ranges for each measured environmental parameters. The said implications corresponding to the judged results includes the messages of recommendations for each said parameter range.

3. An environmental monitoring device according to Claim 1, wherein, the said pre-set built-in standard and criteria includes the second judgment principle. The second judgment principle defines more than one conditional arrays. One or more than one parameter ranges defined by the first judgment principle congregate and become the prerequisite parameter ranges for the establishment of each said conditional array. The said implications corresponding to the judged results includes the messages of potential problems for each said conditional array.

4. An environmental monitoring device according to Claim 3, wherein, the said implications also include the messages of the recommendations, that subjects to the said potential problems.

5. An environmental monitoring device according to Claim 1, wherein, the said pre-set built-in standard and criteria includes the third judgment principle. The said third judgment principle defines more than one categories for each measured environmental parameter. Air-quality-level judgment standards for air quality levels would be defined based on the combination of different categories of the measured environmental parameters. The implications include the messages of the corresponding air quality level by the said air-quality-level judgment standards.

6. An environmental monitoring device according to Claims 1-5, where in, the said sensor could be any sensor of the temperature, relative humidity, volatile organic compounds, carbon monoxide, carbon dioxide, dust, ozone, carbon dioxide, air flow rate, radon, formaldehyde.

7. An environmental monitoring device according to Claims 1-5, where in, The said control unit contains power supply and control circuit, input circuits, the central processing unit, the memory that stored the pre-set built-in standards and criteria for judging the environmental parameters as well as the implications and corresponding messages, and the output circuit. The power supply and control circuit connects the external power supply to the device. The input circuit collects the values from the sensors of different environmental parameters, and output them to the central processing unit. The central processing unit analyzes the input signals base on the pre-set built-in standards and criteria and defines the parameter ranges of each environmental parameter. The implications of each said environmental range are then output and being displayed onto the display unit.

8. An environmental monitoring device according to Claim 7, wherein, the said input circuit includes analog and digital converter and low pulse timer.

9. An environmental monitoring method, which involve steps as follows:
To obtain the value of the environmental parameters;
Judge the value of the environmental parameters against pre-set built-in standard and criteria. Define the parameter ranges of the measured environmental parameters. The implications corresponding to the said parameter ranges are obtained; and
Output the parameter ranges and the implications obtained.
Wherein, the environmental monitoring out analysis and real time data adjustment toward different environmental parameters are carried out, based on their inter-correlationships. An easy-to-understand air quality report is offered instantly.

10. An environmental monitoring method according to Claim 9, wherein, The said pre-set built-in standard and criteria includes the first judgment principle, the second judgment principle and the third judgment principle. The said first judgment principle defines parameter ranges for the value of each measured environmental parameter. The said second judgment principle defines the conditional arrays. One or more than one parameter ranges defined by the first judgment principle congregate and become the prerequisite parameter ranges for the establishment of each said conditional array. The third judgment principle defines more than one categories for each measured environmental parameter Air quality level by the air-quality-level judgment standards would be defined based on the combination of different categories of the measured environmental parameters. The said implications included the messages of the recommendations corresponding to the said parameter ranges, the messages of the potential problems corresponding to the said conditional arrays, the messages of the recommendations corresponding to the said potential problems, and the messages of the corresponding air quality level by the said air-quality-level judgment standards.

11. An environmental monitoring method according to Claim 10, wherein, the said environmental parameters include any parameter of the temperature, relative humidity, volatile organic compounds, carbon monoxide, carbon dioxide, dust, ozone, carbon dioxide, air flow rate, radon, formaldehyde.
